# EUROPEAN PATENT SPECIFICATION

(11) **EP 3 057 694 B1**
(45) Date of publication and mention of the grant of the patent: **15.05.2019**
(21) Application number: 14799546.8
(22) Date of filing: 13.10.2014
(51) Int. Cl.: B01F 5/06, C12M 1/00, C12M 1/12, B01F 11/00, B01J 19/24, B01F 15/06

(54) **APPARATUS FOR MIXING BASED ON OSCILLATORY FLOW REACTORS PROVIDED WITH SMOOTH PERIODIC CONSTRICTIONS**
VORRICHTUNG ZUM MISCHEN AUF DER BASIS VON OSZILLIERENDEN STRÖMUNGSREAKTOREN MIT GLATTEN PERIODISCHEN VERENGUNGEN
APPAREIL DE MÉLANGE BASÉ SUR DES RÉACTEURS À FLUX OSCILLATOIRE DOTÉS D'ÉTRANGLEMENTS PÉRIODIQUES SOUPLES

(30) Priority: 14.10.2013 PT 10723013
(43) Date of publication of application: 24.08.2016
(73) Proprietor: Universidade Do Porto, 4099-002 Porto (PT); Universidade do Minho, 4704-553 Braga (PT)
(72) Inventor: AZEVEDO FERREIRA, António Manuel, 4200-465 Porto (PT); NOGUEIRA DA ROCHA, Fernando Alberto, 4200-465 Porto (PT); COUTO TEIXEIRA, josé António, 4710-057 Braga (PT); MARTINS DE OLIVEIRA SOARES VICENTE, António Augusto, 4710-057 Braga (PT)
(74) Representative: Ferreira, Maria Silvina
(86) International application number: PCT/IB2014/065273
(87) International publication number: WO 2015/056156

(56) References cited:
- WO-A1-2008/151376
- US-A1- 2012 171 090
- ZHENG ET AL: "The axial dispersion performance of an oscillatory flow meso-reactor with relevance to continuous flow operation", CHEMICAL ENGINEERING SCIENCE, OXFORD, GB, vol. 63, no. 7, 17 December 2007 (2007-12-17), pages 1788-1799, XP022524372, ISSN: 0009-2509, DOI: 10.1016/J.CES.2007.12.020 cited in the application
- ZHENG M ET AL: "Biodiesel Reaction Screening Using Oscillatory Flow Meso Reactors", PROCESS SAFETY AND ENVIRONMENTAL PROTECTION, INSTITUTION OF CHEMICAL ENGINEERS, RUGBY, GB, vol. 85, no. 5, 1 January 2007 (2007-01-01), pages 365-371, XP022618629, ISSN: 0957-5820, DOI: 10.1205/PSEP07030 [retrieved on 2007-01-01] cited in the application
- REIS N ET AL: "Residence times and mixing of a novel continuous oscillatory flow screening reactor", CHEMICAL ENGINEERING SCIENCE, OXFORD, GB, vol. 59, no. 22-23, 1 November 2004 (2004-11-01), pages 4967-4974, XP004668405, ISSN: 0009-2509, DOI: 10.1016/J.CES.2004.09.013 cited in the application
- REIS N ET AL: "Liquid backmixing in oscillatory flow through a periodically constricted meso-tube", CHEMICAL ENGINEERING AND PROCESSING, ELSEVIER SEQUOIA, LAUSANNE, CH, vol. 49, no. 7, 1 July 2010 (2010-07-01), pages 793-803, XP027225808, ISSN: 0255-2701 [retrieved on 2010-02-01] cited in the application

## Description

### Technical Field

The present application relates to an apparatus for mixing based on oscillatory flow reactors provided with smooth periodic constrictions.

### Background

Mixing efficiency is the key factor for the success of several processes. Improper mixing can result in non-reproducible processing and lowered product quality. Stirred tank reactor (STR) is commonly used at the industry, however, problems associated with bad mixing, scale up, product quality and process reproducibility, are typically reported. In order to overcome these limitations, associated to the conventional stirred tank reactors, conventional oscillatory flow reactors (OFR) [1,2] and static mixer [3-5] are used. Static mixer is characterized by its small size, intense mixing and enhanced mass and heat transfer. However, as the mixing in these units depends on superficial velocity, the desired mixing is, normally, achieved by increasing the fluid flow or mixer units, a disadvantage in some processes. Unlike static mixers the mixing in OFR can be improved without changing the solution flow and unit numbers, furthermore, it can be operated in batchwise or continuously, since the static mixer can be operated in continuous, flexibility specially relevant to the industry.

OFR is basically a column provided with periodic sharp constrictions, called baffles, operating under oscillatory flow mixing (OFM). The liquid or multiphase fluid is typically oscillated in the axial direction by means of diaphragms, bellows or pistons, at one or both ends of the tube, developing an efficient mixing mechanism where fluid moves from the walls to the center of the tube with intensity controlled by the oscillation frequency (f) and amplitude (*x₀*) [2,6,7]. The formation and dissipation of eddies, in these reactors, has proved to result into significant enhancement in processes such as heat transfer [8], mass transfer [9], particle mixing and separation [10], compared to the continuous stirred tank reactor (CSTR). These singular characteristics place OFR in line with the process intensification that is a major driving force for reactor engineering [11].

In recent years, oscillatory flow reactors (OFRs) have been extensively studied in chemical engineering processes such as crystallization [12,13], polymerization [14-16], fermentation [17] and dispersion [2,18]. Lawton et al. [19] show that continuous crystallization using an oscillatory baffled crystallizer offers significant advantages in terms of process, operation and costs, and delivers the isolation of the model active pharmaceutical ingredient (API) in just over 12 min compared to the 9 h and 40 min in a batch process. Chew and Ristic [12], and Ristic [13], show that the oscillatory baffled batch crystallizer (OBBC) is more effective than the impeller driven batch crystallizer (IDBC) in producing particles of smaller sizes, smoother surfaces and considerably lower crystalline imperfections.

Typically, in order to obtain the best mixing in the OFR the baffle type (annular, spiral, smooth periodic constriction (SPC), etc.), thickness (δ), spacing (L) and open area (α) defined as (orifice diameter (d₀)/ tube diameter (D))², need to be selected and combined with a specific oscillation frequency and amplitude of the fluid. The values of open area (α) are usually disclosed in percentage. A systematic experimental investigation on the effect of baffle free area, baffle spacing and baffle thickness on mixing time in batch oscillatory baffled columns, where D = 50 and 90 mm, provided with annular baffles is reported by Ni et al. [20]. According to the authors the optimal geometrical parameters for obtaining the lowest mixing times in such columns, provided with annular baffles, are: δ=2-3 mm; L= 1.8D; α=20-22%; d₀=0.45-0.50D. Ni Xiongwei [16] claims these values for continuous polymerization using annular baffles and D=40 mm.

Recently, the "conventional" OFR was scaled-down, from the typically 1-15 cm inner diameter to 4.4-5 mm in order to improve the mixing and reduce problems related to the use of annular baffles, linked to the existence of dead zones or stagnant regions near of the baffle which results in several problems of process and product quality. These mesoscale (millilitre) oscillatory baffled reactors (meso-OFR) have received considerable attention due to their mixing intensification, small volume and ability to operate at low flow rates, reducing reagent requirements and waste [21]. Several baffle designs have been tested in order to obtain the best mixing [22-27]. Reis et al. [7,26] redesigned the conventional OFR in order to suit some of the bioprocess applications requirements. The disclosed geometry is based on two concentric tubes, where the inner tube presents Smooth Periodic Constrictions (SPCs), similar to Bellhouse [28], reducing, by this way, the high shear regions that may be crucial to some cell cultures. Flow patterns within this proposed SPC geometry were found to be very dependent of both *x₀* and *f*, as a result of a controlled fluid convection and dispersion within the SPC tube through vortex rings detachment [26,27]. Scale-up studies of the reactor were performed by Zheng and Mackley [29] in order to establish certain process characteristics of the system. The advantages associated with the use of the SPC geometry OFR for biotechnological processes were demonstrated [30,31]. However the application of the SPC design, suggested by Reis et al. [7,26] and Zheng and Mackley [29], to others systems, as crystallization, results in problems related with secondary nucleation, agglomeration and clogging, beyond others. Actually, the OFR based on SPC, hereinafter OFR-SPC, has been restricted to one geometry, two inner diameters and one system. No attention has been paid to studies of SPC geometry change, inner diameter variation, and in the use of this type of geometry and mixing on a possible improvement of the quality of precipitated particles, particularly in crystallization of low output high added-value products, such as pharmaceuticals, dyestuffs, catalysts and proteins. Furthermore, the application of OFR-SPC to multiphase systems is still poorly explored.

### Summary

The present application discloses an apparatus for mixing intensification according to claim 1.

In an embodiment, the reactor vessel of the apparatus is provided with a plurality of inlets or outlets.

In another embodiment, the reactor vessel of the apparatus is in the form of single piece reactor or a plurality of single piece reactors, displaced in parallel, series or both.

In even another embodiment, the reactor vessel of the apparatus is in the form of a single plate reactor or a plurality of plate reactors, displaced in parallel, by stack up the plates.

In an embodiment, the reactor vessel of the apparatus is in the form of a plurality of single piece reactors combined with plate reactors.

In another embodiment, the reactor vessel of the apparatus is totally thermostatized.

In even another embodiment, the jacket on the apparatus is used for mass transfer.

In an embodiment, the mixing chamber of the apparatus is provided with a plurality of ports for inlet or outlet.

According to the invention, the reactor vessel of the apparatus has the convergent - divergent section length (L₁) 1.4 to 2.0 times the inner diameter (D) of the straight section.

According to the invention, the reactor vessel of the apparatus has the shortest diameter (d₀) of the convergent - divergent section 0.41 to 0.6 times the inner diameter (D) of the straight section.

According to the invention, the reactor vessel of the apparatus has the radius of curvature (R_{c}) of the sidewall of the convergent section 0.4 to 0.5 times the inner diameter (D) of the straight section.

According to the invention, the reactor vessel of the apparatus has the radius of curvature (R_{d}) of the sidewall of the divergent section 0.4 to 0.5 times the inner diameter (D) of the straight section.

According to the invention, the reactor vessel of the apparatus has the radius of curvature (Rₜ) at the convergent - divergent section center of the reactor vessel 0.2 to 0.5 times the inner diameter (D) of the straight section.

The present application also disclose the use of the apparatus in multiphase applications such as screening reactions, bioprocess, gas-liquid absorption, liquid-liquid extraction, precipitation and crystallization.

### General description

The present application relates to an apparatus for mixing based on oscillatory flow reactors provided with smooth periodic constrictions. This apparatus can be used in multiphase applications such as screening reactions, bioprocess, gas-liquid absorption, liquid-liquid extraction, precipitation and crystallization.

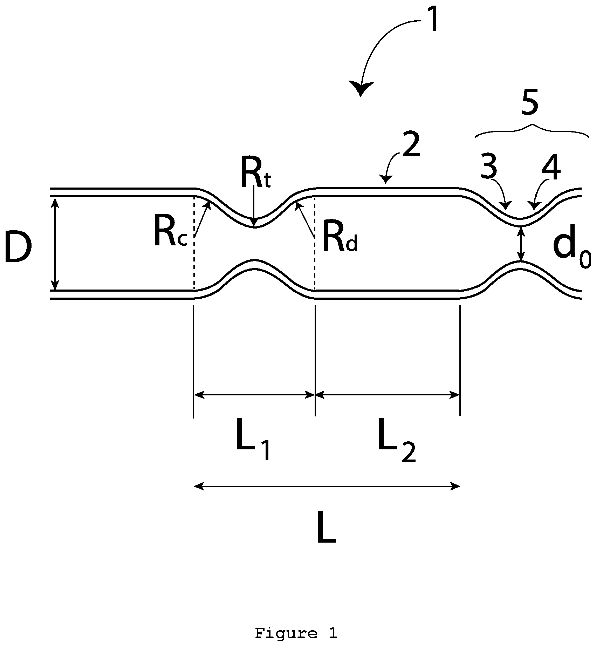

The objective of the technology now disclosed is to provide an improved apparatus for mixing intensification in multiphase systems, which can be operated in continuous or batch mode. So, based on theoretical and experimental observations using different OFR-SPC geometries, as illustrated on Figure 1, and systems, the present technology presents new dimensions ranges that fulfill some of the gaps observed in the "conventional" OFR, as well as in meso-OFR proposed by Reis et al. [7,26] and Zheng and Mackley [29], especially when solids are involved. The geometrical parameters studied were: internal tube diameter (D); internal tube diameter in the constrictions (dₒ); distance (L) between consecutive convergent sections (3); convergent - divergent section (5) length (L₁); straight section (2) length (L₂); radius of curvature (R_{c}) of the sidewall of the convergent section (3); radius of curvature (R_{d}) of the sidewall of the divergent section (4); radius of curvature (Rt) at the convergent - divergent section (5) center; and open area (α), defined as (d₀/D)².

In Table 1, a literature review of OFR dimensions based on SPC and the dimensions of the apparatus disclosed on this application.

**Table 1 - Literature review of OFR dimensions based on SPC.**

| Ref. | L₁ | L | d₀ | R_{c}=R_{d} | Rt | open area (α) |
|---|---|---|---|---|---|---|
| [7,26,27,32 -34] | 1.36D | 2.95D | 0.36D | n/a | n/d | 13% |
| [29] | 1. 2D | 2.6D | 0.40D | n/a | n/d | 16% |
| [35] | n/d | 1-3D | 0.33-0.66D | 0.167-0.5D | n/d | 11-44% |
| Apparatus claimed in the present application | 1.4-2D | 3-4.5D | 0.41-0.60D | 0.4-0.5D | 0.2-0.5D | 17-36% |

The SPC geometries here disclosed decrease the dead zones or stagnant regions, identified in the conventional OFR, and increase its possible use in other systems, as crystallization, decreasing the secondary nucleation, agglomeration and clogging problems, overcoming the gaps of the OFR-SPC geometry presented by Reis et al. [7,26] and Zheng and Mackley [29]. In this application, a dead zone or stagnant region is considered as an area with a low or no mixing.

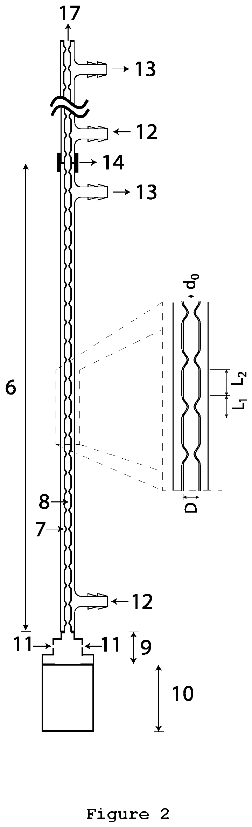

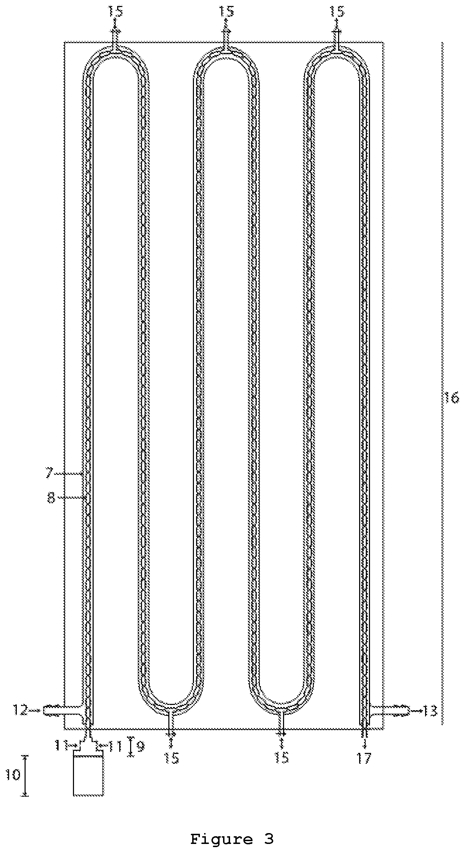

The apparatus that comprises the novel OFR-SPC, based on the claimed dimensions, can be presented as multiple arrangements of: 1) single pieces, as disclosed on Figure 2, in parallel, series, or both; and 2) plates, as disclosed on Figure 3. The plates can be arranged in parallel by stacking up the plates. Single pieces and plates can be combined. This modular system permits the OFR-SPC use in most of the industrial applications. Single pieces and plates are fully thermostatized and can be operated in batchwise or continuously.

In order to provide the fluid oscillation in the OFR-SPC, an oscillatory unit is used.

### Brief description of the Figures

For a better understanding of the technology, some figures are attached representing preferred embodiments of the present technology which, however, are not to be construed as being limiting other possible embodiments falling within the scope of protection.
Figure 1 illustrates a sectional view of the reactor, identifying the design and the parameters that characterize the present technology. In particular, Figure 1 illustrates the following elements:
   1 - Reactor;
   2 - Straight section;
   3 - Convergent section;
   4 - Divergent section;
   5 - Convergent - divergent section;
   D - Inner diameter of the straight section;
   d₀ - Shortest diameter of the convergent - divergent section;
   L - Distance between consecutive convergent sections;
   L₁ - Convergent - divergent section length;
   L₂ - Straight section length;
   R_{c} - Radius of curvature of the sidewall of the convergent section;
   R_{d} - Radius of curvature of the sidewall of the divergent section;
   Rₜ - Radius of curvature at the convergent - divergent section center.
Figure 2 illustrates a plan view of the oscillatory flow reactor apparatus based on single piece reactors. In particular, Figure 2 illustrates the following elements:
   6 - single piece reactor;
   7 - jacket;
   8 - reactor vessel based on SPC;
   9 - mixing chamber;
   10 - oscillatory unit;
   11 - reactor inlet;
   12 - jacket inlet;
   13 - jacket outlet;
   14 - reactor links;
   17 - reactor exit;
   D - Inner diameter of the straight section;
   d₀ - Shortest diameter of the convergent - divergent section;
   L₁ - Convergent - divergent section length;
   L₂ - Straight section length.
Figure 3 illustrates a plan view of the oscillatory flow reactor apparatus based on plate reactor. In particular, Figure 3 illustrates the following elements:
   7 - jacket;
   8 - reactor vessel based on SPC;
   9 - mixing chamber;
   10 - oscillatory unit;
   11 - reactor inlet;
   12 - jacket inlet;
   13 - jacket outlet;
   15 - inlet or outlet;
   16 - plate reactor;
   17 - reactor exit.

### Description of embodiments

The present technology will now be described with reference to the accompanying figures, which however are not to be construed as being limiting other possible embodiments falling within the scope of protection.

The present application relates to an apparatus for mixing based on oscillatory flow reactors provided with smooth periodic constrictions. The present technology comprises new dimensions ranges that characterize the reactor vessel provided with smooth periodic constrictions, here defined as convergent - divergent section (5), and its arrangement in single pieces or plates, as illustrated on Figures 2 and 3.

The reactor vessel (8) may be made of metal, plastic, glass or any porous material. The reactor vessel (8) is characterized by a bundle of reactors (1), as illustrated on Figure 1, that have alternatively straight sections (2) and convergent - divergent sections (5). Each convergent - divergent section (5) consists of a convergent section (3) and a divergent section (4). The convergent section (3) gradually reduces its inner diameter, and the divergent section (4) presents a gradually increasing inner diameter. The shortest inner diameter, obtained at the junction of convergent section (3) and divergent section (4), is defined as d₀. The inner diameter (D) of the straight section (2) is larger than d₀. The convergent and divergent sections have a curved sidewall defined by the radius of curvature (R_{c}) of the sidewall of the convergent section (3), the radius of curvature (R_{d}) of the sidewall of the divergent section (4) and the radius of curvature (Rₜ) at the convergent - divergent section (5) center.

In order to obtain the best mixing condition, the reactor (1) shall fulfill the following conditions:
1. The distance (L) between consecutive convergent sections (3) is 3 to 4.5 times the inner diameter (D) of the straight section (2). That is L = 3-4.5D;
2. The convergent - divergent section (5) length (L₁) is 1.4 to 2 times the inner diameter (D) of the straight section (2). That is L₁ = 1.4-2D;
3. The shortest diameter (d₀) of the convergent - divergent section (5) is 0.41 to 0.6 times the inner diameter (D) of the straight section (2). That is d₀ = 0.41-0.6D;
4. The radius of curvature (R_{c}) of the sidewall of the convergent section (3) is 0.4 to 0.5 times the inner diameter (D) of the straight section (2). That is R_{c} = 0.4-0.5D;
5. The radius of curvature (R_{d}) of the sidewall of the divergent section (4) is 0.4 to 0.5 times the inner diameter (D) of the straight section (2). That is R_{d} = 0.4-0.5D;
6. The radius of curvature (Rₜ) at the convergent - divergent section (5) center is 0.2 to 0.5 times the inner diameter (D) of the straight section (2). That is Rₜ = 0.2-0.5D;
7. The open area (α) takes the values range between 17% and 36%.

The reactor vessel (8) characterized by a bundle of reactors (1) may be incorporated in a single piece reactor (6) or in a plate reactor (16), as illustrated on Figures 2 and 3.

The single piece reactor (6) consists of two concentric tubes, where the inner tube, here defined as reactor vessel (8), presents a bundle of reactors (1), and an external tube used as jacket (7) for reactor vessel (8) thermostatization, or mass transfer, if reactor vessel (8) is made of porous material. The jacket (7) has an inlet (12) and an outlet (13). The single piece reactor (6) can be arranged in parallel, series as shown in Figure 2, or both. The single piece reactors (6) are butt connected by straight tubes. Alternatively, the reactor links (14) can be U tubes. The first single piece reactor (6) is connected to an oscillatory unit (10), which induces a simple harmonic motion to the fluid in the reactor vessel (8), by a mixing chamber (9) provided with several inlets (11).

The plate reactor (16) comprises a continuous serpentine reactor vessel (8), characterized by a bundle of reactors (1), and an external tube used as jacket (7) for reactor vessel (8) thermostatization, or mass transfer, if reactor vessel (8) is made of porous material. The jacket (7) has an inlet (12) and an outlet (13). This reactor vessel (8) has a plurality of inlets or outlets (15), to allow the addition of reactants or other substances, or sample collection. The plate reactor (16) can be arranged in parallel by stacking up the plates. The plate reactors (16) are butt connected by U tubes. The first plate reactor (16) is connected to an oscillatory unit (10), which induces a simple harmonic motion to the fluid in the reactor vessel (8), by a mixing chamber (9) provided with several inlets (11) .

Figure 2 illustrates a plan view of the oscillatory flow reactor apparatus based on single piece reactors (6) characterized by two concentric tubes, where the inner tube, here defined as reactor vessel (8), presents a bundle of reactors (1), and the external tube is used as jacket (7) for reactor vessel (8) thermostatization, or mass transfer if reactor vessel (8) is made of porous material.

Figure 3 shows a plan view of the oscillatory flow reactor apparatus based on plate reactor (16), constituted by an inner tube, here defined as reactor vessel (8), presenting a bundle of reactors (1), an external tube used as jacket (7) for reactor vessel (8) thermostatization, or mass transfer if reactor vessel (8) is made of porous material, and several inlets or outlets (15), to allow the addition of reactants or other substances, or sample collection.

Single piece reactors (6) and plate reactors (16) can be closed using a close valve at reactor exit (17).

Single piece reactors (6) and plate reactors (16) can be combined.

The number, size and length of the single piece reactor (6), plate reactor (16) or single piece reactor (6) and plate reactor (16) combinations are designed according to the system specification.

Single piece reactors (6) and plate reactors (16) can be operated in batchwise or continuously.

The liquid or multiphase fluids are fed to the reactor vessel (8) through the inlets (11) of the mixing chamber (9) .

The liquid or multiphase fluid is oscillated in the axial direction by means of oscillatory unit (10), developing an efficient mixing mechanism where fluid moves from the walls to the center of the tube with intensity controlled by the oscillation frequency (*f*) and amplitude (*x₀*). The formation and dissipation of eddies in the reactor results into significant enhancement in processes such as heat transfer, mass transfer, particle mixing and separation, beyond others.

The reactor will obtain the optimum mixing conditions when:
1. The distance (L) between consecutive convergent sections (3) is 3 to 4.5 times the inner diameter (D) of the straight section (2), preferably 3.25D;
2. The convergent - divergent section (5) length (L₁) is 1.4 to 2 times the inner diameter (D) of the straight section (2), preferably 1.5D;
3. The shortest diameter (d₀) of the convergent - divergent section (5) is 0.41 to 0.6 times the inner diameter (D) of the straight section (2), preferably 0.42D;
4. The radius of curvature (R_{c}) of the sidewall of the convergent section (3) is 0.4 to 0.5 times the inner diameter (D) of the straight section (2), preferably 0.47D;
5. The radius of curvature (R_{d}) of the sidewall of the divergent section (4) is 0.4 to 0.5 times the inner diameter (D) of the straight section (2), preferably 0.47D;
6. The radius of curvature (Rₜ) at the convergent - divergent section (5) center is 0.2 to 0.5 times the inner diameter (D) of the straight section (2), preferably 0.32D;
7. The open area (α) takes the values range between 17% and 36%, preferably, 18%;
8. The oscillation frequency of the medium is between 1 and 6 Hz;
9. The oscillation amplitude of the medium is between 0 and 0.5 times the distance (L) between consecutive convergent sections (3).

The disclosed technology can be used in mass and heat transfer intensification. In particular, the disclosed technology can be used in mixing intensification between liquid/liquid, liquid/gas and liquid/solid phases.

The disclosed technology overcome the disadvantages of the conventional OFR, based on annular baffles, especially in what concerns the dead zones decreasing and the quick cleaning process. The disclosed technology also overcome the disadvantages of the meso-OFR based on SPC, especially in what concerns the decrease of the secondary nucleation, agglomeration and clogging problems.

As the disclosed technology is based on a modular system, it allows a quick reactor change according to the industries needs, a distinguishing and striking characteristic of other reactors.

The disclosed technology can be operated in batchwise or continuously, this characteristic being of particular relevance in chemical, bio-chemical, biological and pharmaceutical industry.

The disclosed technology offers unique features in comparison with conventional chemical reactors. It is suitable for multiphase applications such as screening reactions, bioprocess, gas-liquid absorption, precipitation and crystallization operating in batch or continuous mode. As example: crystallization process - this technology offers a metastable zone increase, a better supersaturation control and a narrow crystal size distribution in crystallization of active pharmaceutical ingredients such as paracetamol and proteins such as lysozyme, beyond others; precipitation - a mixing intensification of the solution from the first contact moments, that results in a single species precipitation without the presence of others contaminant species typical of bad mixing and always present in others reactors, was observed in the hydroxyapatite (Hap) precipitation process; gas-liquid systems - a significant mass transfer increase, up to ten-fold, in comparison with conventional reactors was observed; liquid-liquid extraction - significant increase of the contact area and an uniform drops distribution inside of the reactor when compared with conventional reactors was observed.

Some tests were made using the technology now disclosed using different systems and OFR-SPC arrangements:
- gas-liquid systems: the mass transfer coefficient (kLa) values obtained with the novel OFR, as illustrated in Figure 2, represented a up to ten-fold increase in comparison with a bubble column and up to eight-fold increase in comparison with the conventional OFR;
- liquid-liquid extraction: a good performance in liquid-liquid mixing was observed. It is possible to control the drop size varying the oscillation frequency and amplitude. A significant increase of the contact area was observed;
- liquid-solid system: particles can be well suspended in a volume concentration up to 40%;
- crystallization: a continuous crystallization of hydroxyapatite (Hap) was successfully obtained. Using the apparatus, as illustrated in Figure 3, it was possible to obtain Hap with high chemical purity. A decrease up to 75% of the time required to obtain the crystals, when compared with the common crystallizers, was also verified. Furthermore, it has been shown that the mean particle size and the aggregation degree of the prepared HAp particles can be controlled by changing the residence time of the solution in the reactor.

These results show the capability of the present apparatus, operating in continuous or batch mode, for mixing intensification using multiphase systems.

### References

[1] M.R. Mackley, R.L. Skelton, K.B. Smith, Processing of liquid/solid mixtures using pulsations, GB 2 276 559 A, 1994.
[2] X. Ni, K.. Murray, Y. Zhang, D. Bennett, T. Howes, Polymer product engineering utilising oscillatory baffled reactors, Powder Technol. 124 (2002) 281-286. doi:10.1016/S0032-5910(02)00022-0.
[3] R.K. Thakur, C. Vial, K.D.P. Nigam, E.B. Nauman, G. Djelveh, Static mixers in the process industries -a review, Chem. Eng. Res. Des. 81 (2003).
[4] J.C.B. Lopes, P. Laranjeira, M. Dias, A. Martins, Network mixer and related mixing process, US 2009/0016154 A1, 2009.
[5] P.E.M.S.C. Laranjeira, NETMIX Static Mixer - Modelling, CFD simulation and Experimental Characterisation, Faculdade de Engenharia, Universidade do Porto, 2005.
[6] X. Ni, H. Jian, A.W. Fitch, Computational fluid dynamic modelling of flow patterns in an oscillatory baffled column, Chem. Eng. Sci. 57 (2002) 2849-2862.
[7] N.M.F. Reis, Novel Oscillatory Flow Reactors for Biotechnological Applications, Minho University, 2006.
[8] M. Mackley, P. Stonestreet, Heat-Transfer and Associated Energy Dissipation for Oscillatory Flow in Baffled Tubes, Chem. Eng. Sci. 50 (1995) 2211-2224.
[9] A.W. Fitch, H. Jian, X. Ni, An investigation of the effect of viscosity on mixing in an oscillatory baffled column using digital particle image velocimetry and computational fluid dynamics simulation, Chem. Eng. J. 112 (2005) 197-210. doi:10.1016/j.cej.2005.07.013.
[10] M. Mackley, K. Smith, N. Wise, The Mixing and Separation of Particle Suspensions Using Oscillatory Flow in Baffled Tubes, Chem. Eng. Res. Des. 71 (1993) 649-656.
[11] M. Mackley, Process and product innovation, J. Chem. Technol. Biotechnol. 78 (2003) 94-97.
[12] C.M. Chew, R.I. Ristic, Crystallization by oscillatory and conventional mixing at constant power density, AIChE J. 51 (2005) 1576-1579. doi:10.1002/aic.10391.
[13] R.I. Ristic, Oscillatory Mixing for Crystallization of High Crystal Perfection Pharmaceuticals, Chem. Eng. Res. Des. 85 (2007) 937-944. doi:10.1205/cherd06235.
[14] X. Ni, D.C. Bennett, K.C. Symes, B.D. Grey, Inverse Phase Suspension Polymerization of Acrylamide in a Batch Oscillatory Baffled Reactor, Polymer (Guildf). (1999) 1669-1676.
[15] X. Ni, J.C. Johnstone, C.S. Chemicals, A. Div, W.T. Limited, B. Bd, Suspension Polymerization of Acrylamide in an Oscillatory Baffled Reactor: from Drops to Particles, AIChE J. 47 (2001) 1746-1757.
[16] X. Ni, Method and apparatus for phase separated synthesis, US 6,429,268 B1, 2002.
[17] X. Ni, G. S, R. Cumming, P. DW, A Comparative-Study of Mass-Transfer in Yeast for a Batch Pulsed Baffled Bioreactor and a Stirred-Tank Fermenter, Chem. Eng. Sci. 50 (1995) 2127-2136.
[18] A. Harvey, M.R. Mackley, N. Reis, J.A. Teixeira, A.A. Vicente, Fluid mixing and particle suspension in a novel microreactor, in: Proceeding 30th Conf. SSCHE, 2003: pp. 26-30.
[19] S. Lawton, G. Steele, P. Shering, L. Zhao, I. Laird, X.-W. Ni, Continuous Crystallization of Pharmaceuticals Using a Continuous Oscillatory Baffled Crystallizer, Org. Process Res. Dev. 13 (2009) 1357-1363.
[20] X. Ni, G. Brogan, A. Struthers, D.C. Bennett, S.F. Wilson, A SYSTEMATIC STUDY OF THE EFFECT OF GEOMETRICAL PARAMETERS ON MIXING TIME IN OSCILLATORY BAFFLED COLUMNS, Chem. Eng. Res. Des. 76 (1998) 635-642.
[21] A.N. Phan, A. Harvey, J. Lavender, Characterisation of fluid mixing in novel designs of mesoscale oscillatory baffled reactors operating at low flow rates (0.3-0.6ml/min), Chem. Eng. Process. Process Intensif. 50 (2011) 254-263. doi:10.1016/j.cep.2011.02.004.
[22] A.N. Phan, A. Harvey, Development and evaluation of novel designs of continuous mesoscale oscillatory baffled reactors, Chem. Eng. J. 159 (2010) 212-219. doi:10.1016/j.cej.2010.02.059.
[23] A.N. Phan, A.P. Harvey, M. Rawcliffe, Continuous screening of base-catalysed biodiesel production using New designs of mesoscale oscillatory baffled reactors, Fuel Process. Technol. 92 (2011) 1560-1567. doi:10.1016/j.fuproc.2011.03.022.
[24] A.N. Phan, A.P. Harvey, Effect of geometrical parameters on fluid mixing in novel mesoscale oscillatory helical baffled designs, Chem. Eng. J. 169 (2011) 339-347. doi:10.1016/j.cej.2011.03.026.
[25] A.N. Phan, A.P. Harvey, Characterisation of mesoscale oscillatory helical baffled reactor-Experimental approach, Chem. Eng. J. 180 (2012) 229-236. doi:10.1016/j.cej.2011.11.018.
[26] N. Reis, a. a. Vicente, J. a. Teixeira, M.R. Mackley, Residence times and mixing of a novel continuous oscillatory flow screening reactor, Chem. Eng. Sci. 59 (2004) 4967-4974. doi:10.1016/j.ces.2004.09.013.
[27] N. Reis, A. Harvey, M. Mackley, A. Vicente, J. Teixeira, Fluid Mechanics and Design Aspects of a Novel Oscillatory Flow Screening Mesoreactor, Chem. Eng. Res. Des. 83 (2005) 357-371. doi:10.1205/cherd.03401.
[28] B. Bellhouse, Method for effecting heat or mass transfer, US4075091A, 1978.
[29] M. Zheng, M. Mackley, The axial dispersion performance of an oscillatory flow meso-reactor with relevance to continuous flow operation, Chem. Eng. Sci. 63 (2008) 1788-1799. doi:10.1016/j.ces.2007.12.020.
[30] N. Reis, C.N. Gonc, A.A. Vicente, J.A. Teixeira, Proof-of-Concept of a Novel Micro-Bioreactor for Fast Development of Industrial Bioprocesses, Biotechnol. Bioeng. 95 (2006) 744-753. doi:10.1002/bit.
[31] M. Zheng, R.L. Skelton, M.R. Mackley, BIODIESEL REACTION SCREENING USING OSCILLATORY FLOW MESO REACTORS, Trans IChemE, Part B, Process Saf. Environ. Prot. 85 (2007) 365-371.
[32] N. Reis, R.N. Pereira, A.A. Vicente, J.A. Teixeira, Enhanced Gas-Liquid Mass Transfer of an Oscillatory Constricted-Tubular Reactor, Ind. Eng. Chem. Res. 47 (2008) 7190-7201.
[33] N. Reis, P.C. Mena, A. a. Vicente, J. a. Teixeira, F. a. Rocha, The intensification of gas-liquid flows with a periodic, constricted oscillatory-meso tube, Chem. Eng. Sci. 62 (2007) 7454-7462. doi:10.1016/j.ces.2007.09.018.
[34] N. Reis, a. a. Vicente, J. a. Teixeira, Liquid backmixing in oscillatory flow through a periodically constricted meso-tube, Chem. Eng. Process. Process Intensif. 49 (2010) 793-803. doi:10.1016/j.cep.2010.01.014.
[35] R.-H. Chang, CONTINUOUS TUBULAR FLOW REACTOR AND CORRUGATED REACTOR TUBE FOR THE REACTOR, US 2012/0171090 A1, 2012.

The description, of course, is in no way limited to the embodiments described in this document and any person skilled in the art may envisage many possibilities of modifying it, sticking to the general idea, as defined in the claims.

## Claims

1. An apparatus for mixing intensification comprising:
- a reactor vessel provided with Smooth Periodic Constrictions wherein the distance (L) between consecutive convergent sections is 3 to 4.5 times the inner diameter (D) of the straight section, the convergent-divergent section length (L₁) of the reactor vessel is 1.4 to 2.0 times the inner diameter (D) of the straight section, the shortest diameter (d₀) of the convergent- divergent section of the reactor vessel is 0.41 to 0.6 times the inner diameter (D) of the straight section, the radius of curvature (R_{c}) of the sidewall of the convergent section of the reactor vessel is 0.4 to 0.5 times the inner diameter (D) of the straight section, the radius of curvature (R_{d}) of the sidewall of the divergent section of the reactor vessel is 0.4 to 0.5 times the inner diameter (D) of the straight section, the radius of curvature (Rₜ) at the convergent-divergent section center of the reactor vessel is 0.2 to 0.5 times the inner diameter (D) of the straight section, and the open area (α) defined as (d₀/D)² is between 17 and 36%;
- a mixing chamber;
- oscillation means to oscillate a system within the reactor vessel.

2. An apparatus according to the previous claim, wherein said reactor vessel is provided with a plurality of inlets or outlets.

3. An apparatus according to any of the previous claims, wherein said reactor vessel is in the form of a single piece reactor or a plurality of single piece reactors, displaced in parallel, series or both.

4. An apparatus according to any of the previous claims, wherein said reactor vessel is in the form of a single plate reactor or a plurality of plate reactors, displaced in parallel, by stacking up the plates.

5. An apparatus according to any of the previous claims, wherein said reactor vessel is in the form of a plurality of single piece reactors combined with plate reactors.

6. An apparatus according to any of the previous claims, wherein said reactor vessel is totally thermostatized.

7. An apparatus according to any of the previous claims, wherein a jacket is used for mass transfer.

8. An apparatus according to any of the previous claims, wherein the mixing chamber is provided with a plurality of ports for inlet or outlet.

9. Use of the apparatus disclosed in any one of the previous claims in multiphase applications such as screening reactions, bioprocess, gas-liquid absorption, liquid-liquid extraction, precipitation and crystallization.

## Patentansprüche

1. Ein Gerät zur Mischungsintensivierung, bestehend aus:
- einem mit regelmäßigen glatten Einengungen versehenen Reaktorbehälter, wobei der Abstand (L) zwischen den aufeinander folgenden konvergierenden Abschnitten 3 bis 4,5 mal größer ist als der Innendurchmesser (D) des geraden Abschnitts, die Länge (L₁) des konvergierenden - divergierenden Abschnitts des Reaktorbehälters 1,4 bis 2,0 mal größer ist als der Innendurchmesser (D) des geraden Abschnitts, der kleinste Durchmesser (d₀) des konvergierenden - divergierenden Abschnitts des Reaktorbehälters 0,41 bis 0,6 mal kleiner ist als der Innendurchmesser (D) des geraden Abschnitts, der Krümmungsradius (R_{c}) der Seitenwand des konvergierenden Abschnitts des Reaktorbehälters 0,4 bis 0,5 mal kleiner ist als der Innendurchmesser (D) des geraden Abschnitts, der Krümmungsradius (R_{d}) der Seitenwand des divergierenden Abschnitts des Reaktorbehälters 0,4 bis 0,5 mal kleiner ist als der Innendurchmesser (D) des geraden Abschnitts, der Krümmungsradius (Rₜ) in der Mitte des konvergierenden - divergierenden Abschnitts des Reaktorbehälters 0,2 bis 0,5 mal kleiner ist als der Innendurchmesser (D) des geraden Abschnitts und der als (d₀/D)² definierte offene Bereich (a) zwischen 17 und 36% beträgt.
- einer Mischkammer;
- einem Oszillationsmittel, um ein System innerhalb des Reaktorbehälters zu oszillieren.

2. Ein Gerät gemäß dem vorherigen Anspruch, wobei der besagte Reaktorbehälter mit einer Vielzahl von Einlässen oder Auslässen ausgerüstet ist.

3. Ein Gerät gemäß einem der vorherigen Ansprüche, wobei der besagte Reaktorbehälter in Form eines einzelnen Reaktors oder einer Vielzahl von einzelnen Reaktoren existiert, die parallel, in Reihe oder beidseitig angeordnet sind.

4. Ein Gerät gemäß einem der vorherigen Ansprüche, wobei der besagte Reaktorbehälter in Form eines einzelnen Plattenreaktors oder einer Vielzahl von parallel angeordneten Plattenreaktoren existiert, die durch das Stapeln der Platten erreicht wird.

5. Ein Gerät gemäß einem der vorherigen Ansprüche, wobei der besagte Reaktorbehälter in Form einer Vielzahl von einzelnen Reaktoren vorhanden ist, die mit Plattenreaktoren kombiniert werden.

6. Ein Gerät gemäß einem der vorherigen Ansprüche, wobei der besagte Reaktorbehälter vollkommen thermostatisiert ist.

7. Ein Gerät gemäß einem der vorherigen Ansprüche, wobei ein Mantel zum Stofftransfer verwendet wird.

8. Ein Gerät gemäß einem der vorherigen Ansprüche, wobei die Mischkammer mit einer Vielzahl von Anschlüssen zum Einlass oder Auslass ausgerüstet ist.

9. Verwendung des in einem der vorherigen Ansprüche aufgezeigten Geräts in Mehrphasen-Anwendungen, wie beispielsweise Screening-Reaktionen, Bioprozessen, Gas-Flüssigkeitsaufnahme, Flüssig-Flüssig-Extraktion, Ausscheidung und Kristallisation.

## Revendications

1. Un appareil d'intensification de mélange comprenant :
- une cuve de réacteur dotée de Constrictions Périodiques Douces dans laquelle la distance (L) entre les sections convergentes consécutives est de 3 à 4,5 fois le diamètre intérieur (D) de la section rectiligne, la longueur de section convergente - divergente (L₁) de la cuve de réacteur est de 1,4 à 2,0 fois le diamètre intérieur (D) de la section rectiligne, le diamètre le plus petit (d₀) de la section convergente - divergente de la cuve du réacteur est de 0,41 à 0,6 fois le diamètre intérieur (D) de la section rectiligne, le rayon de courbure (R_{c}) de la paroi latérale de la section convergente de la cuve du réacteur est de 0,4 à 0,5 fois le diamètre intérieur (D) de la section rectiligne, le rayon de courbure (R_{d}) de la paroi latérale de la section divergente de la cuve du réacteur est de 0,4 à 0,5 fois le diamètre intérieur (D) de la section rectiligne, le rayon de courbure (Rₜ) au centre de la section convergente - divergente de la cuve du réacteur est de 0,2 à 0,5 fois le diamètre intérieur (D) de la section rectiligne, et l'aire ouverte (α) définie comme (do/ D)² est comprise entre 17 et 36%
- une chambre de mélange ;
- des moyens d'oscillation pour faire osciller un système dans la cuve du réacteur.

2. Appareil selon la revendication précédente, dans lequel ladite cuve de réacteur est fournie avec une pluralité d'entrées ou de sorties.

3. Appareil selon l'une quelconque des revendications précédentes, dans lequel ladite cuve de réacteur se présente sous la forme d'un réacteur monobloc ou d'une pluralité de réacteurs monoblocs, déplacés en parallèle, en série ou les deux.

4. Appareil selon l'une quelconque des revendications précédentes, dans lequel ladite cuve de réacteur se présente sous la forme d'un réacteur à plaque unique ou d'une pluralité de réacteurs à plaque, déplacés en parallèle, par empilage des plaques.

5. Appareil selon l'une quelconque des revendications précédentes, dans lequel ladite cuve de réacteur se présente sous la forme d'une pluralité de réacteurs monoblocs combinés à des réacteurs à plaque.

6. Appareil selon l'une quelconque des revendications précédentes, dans lequel ladite cuve de réacteur est totalement thermostatisée.

7. Appareil selon l'une quelconque des revendications précédentes, dans lequel une enveloppe est utilisée pour le transfert de masse.

8. Appareil selon l'une quelconque des revendications précédentes, dans lequel la chambre de mélange est munie d'une pluralité d'orifices pour l'entrée ou la sortie.

9. Utilisation de l'appareil décrit dans l'une quelconque des revendications précédentes dans des applications multiphases telles que des réactions de criblage, un bioprocédé, une absorption gaz - liquide, une extraction liquide - liquide, une précipitation et une cristallisation.
